# EUROPEAN PATENT SPECIFICATION

(11) **EP 2 101 742 B1**
(45) Date of publication and mention of the grant of the patent: **11.05.2011**
(21) Application number: 07824998.4
(22) Date of filing: 13.11.2007
(51) Int. Cl.: A61K 9/20, A61K 9/28

(54) **PHARMACEUTICAL COMPOSITION CONTAINING CLOPIDOGREL HYDROGENESULPHATE OF POLYMORPH 1 FORM**
CLOPIDOGREL-HYDROGENSULFAT VON POLYMORPH 1 FORM ENTHALTENDE PHARMAZEUTISCHE ZUSAMMENSETZUNG
COMPOSITION PHARMACEUTIQUE CONTENANT DE L'HYDROGÉNOSULFATE DE CLOPIDOGREL DE FORME POLYMORPHE 1

(30) Priority: 14.11.2006 HU 0600839
(43) Date of publication of application: 23.09.2009
(73) Proprietor: Egis Gyógyszergyár Nyilvánosan Müködö, 1106 Budapest (HU)
(72) Inventor: FEKETE, Pál, 1147 Budapest (HU); SZLÁVYNÉ SZÉLL, Zsuzsa, 1106 Budapest (HU); SZABÖNÉ RÉVÉSZ, Piroska, 6726 Szeged (HU); ZSIGMOND, Zsolt, 1173 Budapest (HU); LEVENTISZNÉ HUSZÁR, Magdolna, 1125 Budapest (HU); PÁLFI, Zoltánné, 1172 Budapest (HU); BURESNÉ PAPP, Cecilla, 2100 Gödöllö (HU)
(74) Representative: Beszédes, Stephan G.
(86) International application number: PCT/HU2007/000106
(87) International publication number: WO 2008/059298

(56) References cited:
- EP-A- 1 310 245
- EP-A- 1 674 468
- WO-A-03/051362
- WO-A-2005/070464
- FR-A- 2 792 836

## Description

### FIELD OF THE INVENTION

The present invention relates to pharmaceutical compositions containing polymorph 1 form (***S***)-(+)-(2-chlorophenyl)-2-(6,7-dihydro-4*H*-thieno[3,2-c]pyridine-5-yl-acetate hydrogen sulphate (INN name clopidogrel hydrogene sulphate) of the formula

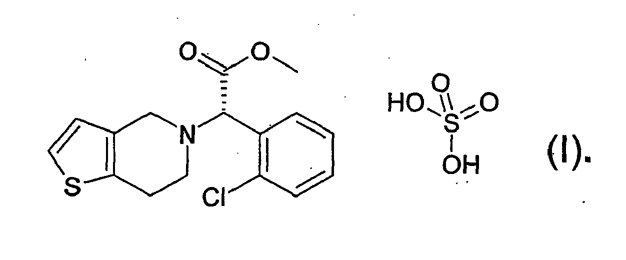

These pharmaceutical compositions inhibit the formation of plaques in arteries and the evolution of arteriosclerosis. The chemical formula of the clopidogrel hydrogen sulphate is C₁₆H₁₆ClNO₂S*H₂SO₄. The active ingredient is white or almost white powder having a very low solubility in water at neutral pH but good solubility in aqueous acidic conditions. The solubility of the compound in methanol and ethanol is also good, but in ether is low. Several crystalline polymorph forms and the amorphous form of the clopidogrel hydrogen sulphate are known.

Particularly, the present invention relates to pharmaceutical compositions containing crystalline polymorph 1 form of clopidogrel hydrogensulphate, other auxiliary and filling agents, which result in the long term chemical and crystallographic stability of the sensitive active ingredient. Pharmaceutical compositions according to the present invention have very good dissolution rate even after a long time storage.

### TECHNICAL BACKGROUND OF THE INVENTION

The different polymorph forms usually have different physical and stability properties. From the point of view of the chemical stability and the ageing of the pharmaceutical composition the selection of the suitable polymorph form is crucial.

In case of the clopidogrel hydrogensulphate several polymorph forms are known. The polymorphism of clopidogrel was mentioned first in the description of the international patent application No. WO 99/65915. Polymorph forms 1 and 2 are distinguished. Polymorph form 2 is mentioned as a new polymorph form and polymorph form 1 is identified by its melting point as the polymorph form disclosed in the description of the European patent No. EP 281 459.

The recently marketed product contains the thermodynamically more stabile polymorph form 2 according to X ray diffraction examinations.

In the international patent application No. WO 03/051362 the amorphous form and polymorph III, IV, V, VI forms are disclosed.

The amorphous form and the different polymorph forms have different thermic properties, melting points, differential caloric (DSC), thermogravimetric properties, X-ray diffraction and infrared data. Based on these data the different polymorph forms can be well distinguished from each other.

These polymorph forms have very different solubility, flowability and compressibility as well. These properties are very important and have to be taken into consideration during the design of new pharmaceutical formulations.

The dissolution rate of the amorphous form and the different polymorph forms in aqueous media is very important. The solubility of the active ingredient subsequent to the administration of the pharmaceutical composition can influence the therapeutic effect too. The low solubility rate can reduce the absorption rate or the total amount of the absorbed pharmaceutical active ingredient. Usually, the thermodynamically less stable polymorph forms have higher solubility rates.

The dissolution rate of the pharmaceutical active ingredient from the pharmaceutical composition depends besides of its polymorph form on the composition of the formula and the changes occurred during of tablet compression and storage.

The less chemical stability of the polymorph forms having higher solubility is a disadvantage, therefore in case of using these polymorph forms the compositions, which were elaborated for using the more stable polymorph form, usually have to be reformulated.

Wet granulation is not applicable when using these polymorphs because of the recrystallization of the active ingredient, which can happen very easily during the wetting process with the consequence that the less soluble polymorph form is formed. The chemical stability of the used clopidogrel hydrogensulphate is crucial because its incompatibility with several auxiliary agents as magnesium stearate, povidone, gelatine is known. In the presence of these compounds, the formation of degradation products is faster. The presence of humidity in the pharmaceutical composition also causes faster degradation of clopidogrel.

Neither in the description of the above-mentioned international patent application No. 99/65915, nor in the description of the international patent application No. WO 03/0513915 are there disclosed formulations of the pharmaceutical compositions using different polymorph forms, nor is there any teaching about the stability of the pharmaceutical compositions thereof.

Several polymorph forms are disclosed in the international patent application No. WO 03/051362, furthermore there are disclosed auxiliary agents and fillers in detail which are applicable for the preparation of solid or fluid dosage forms. The teaching is only a mere listing of auxiliary agents and fillers. There is no hint about the way for the preparation of stable pharmaceutical compositions and the selection of the suitable auxiliary agents, fillers, new or known polymorph forms. Some listed compounds, e.g. magnesium or calcium stearate can cause the degradation of the active ingredient to a possible extent of 3.8% in the pharmaceutical composition according to the earlier published European patent application No. EP 1 310 245. Furthermore, the international patent application No. 03/051362 neither contains any stability data about the pharmaceutical compositions nor examples for the preparation thereof.

The active ingredients having a similar dihydrothien[3,2]pyridyl chemical structure are very sensitive. The chemical structure of ticlodipine of the formula 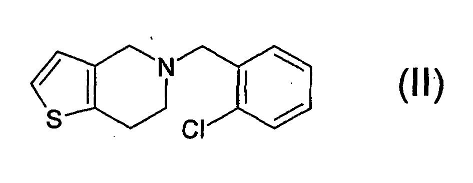 differs from the clopidogrel only that a hydrogen atom substitutes the methoxycarbonyl group of clopidogrel. There are several inventions dealing with pharmaceutical compositions containing ticlopidine. These descriptions disclose the improper auxiliary and filling agents. According to the inventors of the US patent No. 5,520,928 ticlopidine and similar compounds decompose unexpectedly fast in the presence of some well known excipients like gelatine, povidone or magnesium stearate. According to the description of the international patent application No. WO 00/01364 the use of sodium starch glycolate also leads to the decomposition

of the active ingredient. According to this invention stable pharmaceutical compositions can be prepared by using hydrophilic tenzides, e.g. polyethylene glycols and acceptable excipients. Therefore it is not surprising that stability problems have appeared at the pharmaceutical compositions containing similar pharmaceutical active ingredient, namely clopidogrel. Some technical solutions are suggested in the literature to improve the storage stability of the pharmaceutical compositions containing clopidogrel.

According to the description of the European patent application No. 1 310 245 the use of zinc stearate or sodium stearyl fumarate is suggested instead of magnesium stearate because the lubricant activity of hydrogenated castor oil used in the marketed composition was found inappropriate.

In the description of the international patent application No. WO 2005/070464 hydrogenated vegetable oils are used instead of magnesium stearate to improve the stability of tablets and the use of sodium carboxymethyl starch is suggested to solve tabletting problems. The use of hydrogenated vegetable oils to improve the stability is known from the literature, moreover it was already described in case of the marketed composition. There are, however, no data in the application about the improvement resulted from the use of a higher amount of lubricants than usual compared to the known compositions.

The above-mentioned patent applications describe neither polymorph forms used nor the decrease of the dissolution rate of clopidogrel during the long time storage of the composition.

Recently the pharmaceutical composition is marketed in a form of film tablet, which contains 97.9 mg of clopidogrel hydrogensulphate in form of polymorph 2 corresponding to 75 mg of clopidogrel base. The composition was elaborated with respect to the incompatibilities mentioned above. One of the less hygroscopic agent, mannitol is used as filling agent (its equilibrium moisture content is 0.3% at 75 % relative humidity). Hydroxypropyl cellulose, an inert, ether type agent is used as disintegrant. An ester type lubricant, hydrogenated castor oil (Cutina H) and ether and alcohol type polyethyleneglycol are used as lubricant. As further excipient the composition contains microcrystalline cellulose, and the coating contains hydroxypropylmethyl cellulose, triacetin, carnauba wax, titan dioxide an ferrous oxide pigments.

The fast dissolution of clopidogrel hydrogensulphate is very important. To improve the dissolution rate the use of less stable polymorph forms having better dissolution properties is more suitable.

The inventors of the Hungarian patent application No. P 04 02633 disclose an amorphous form of clopidogrel hydrogensulphate. From the point of view of dissolution a composition containing this form is very advantageous. However, the preparation of the solid dispersion has some disadvantages from technological point of view.

According to the invention the clopidogrel hydrogen sulphate is dissolved in a suitable organic solution, then colloid or microcrystalline cellulose is suspended in the solution, or the solution is applied on the surface of microcrystalline cellulose and the solvent is evaporated.

The use of the organic solvents and the evaporation in fluid-bed apparatus are very disadvantageous from both environmental and technological points of view. A further disadvantage is that the final composition can be achieved after several subsequent technological steps.

The stabilities of the polymorph forms mentioned above are not known. According to the process disclosed in the description of European patent No. 281459 the polymorph form 1 is obtained. This labile polymorph form in a suitable environment keeps its chemical purity and polymorph form until the formulation.

The storage stability is very important, because as it is known by a person skilled in the art, the more labile polymorph form of active ingredients is ready to transform into its more stable polymorph form during the long storage. This transformation can occur during the formulation or the storage and causes the change of the dissolution properties of the composition.

Our purpose was to develop a stable pharmaceutical composition containing clopidogrel hydrogensulphate of polymorph form 1 in which the active ingredient keeps its chemical stability and morphologic form, and the dissolution properties meet the requirements of pharmacopoeias even after a long storage period. Our further purpose was to avoid the use of organic solvents in the process and find the less complicated technological process for the preparation of the said composition.

### SUMMARY OF THE INVENTION

The present invention provides a pharmaceutical composition containing clopidogrel hydrogensulphate of polymorph 1 form as active ingredient, microcrystalline cellulose and colloid silica or if necessary silanized microcrystalline cellulose as filling and binding agent, cellulose ether type compound as disintegrating agent and a fatty acid ester type compound as gliding agent.

### DETAILED DESCRIPTION OF THE INVENTION

We found surprisingly that the dissolution rates of pharmaceutical compositions containing the ingredients disclosed above has not reduced notwithstanding that the European patent No. 1310245 discloses that the microcrystalline cellulose reduces the dissolution rate of clopidogrel hydrogensulphate from the composition. In the pharmaceutical composition according to the present invention the polymorph form 1 does not transform into polymorph form 2. Furthermore, the chemical stability of the active ingredient is also very good. It is surprising that the relatively high microcrystalline cellulose content does not reduce the chemical and crystallographic stability of the composition. The microcrystalline cellulose and the colloid silica are so-called hygroscopic compounds. The equilibrium moisture content of microcrystalline cellulose is approximately 8 %, the equilibrium moisture content of colloid silica is approximately 15 % at 75 % relative humidity. Based on these facts, in case of the mixtures of clopidogrel hydrogensulphate of polymorph form 1 with microcrystalline cellulose and colloid silica much more a higher decomposition and crystallographic transformation can be expected - due to the high humidity content - than a higher chemical and crystallographic stability.

These surprising facts are supported by Table 1 showing the stability data of the pharmaceutical composition according to Example 1 containing the clopidogrel hydrogen sulphate polymorph form 1 as follows:

**Table 1**

| Impurities of the filmcoated tablets according to the present invention: | | | | | | |
|---|---|---|---|---|---|---|
| Film tablet according to present invention | Product after the preparation process | | After 3 months; 40°C, 75% humidity | | After 6 months; 40°C, 75% humidity | |
| Charge No. | 361104 | 391204 | 361104 | 391204 | 361104 | 391204 |
| 4-oxo-thienopyridine | < 0.01 | < 0.01 | 0.01 | < 0.01 | 0.01 | < 0.01 |
| Free acid | < 0.01 | < 0.01 | 0.102 | 0.086 | 0.171 | 0.16 |
| Impurity-3 | 0.1 | 0.27 | 0.029 | 0.029 | 0.029 | 0.029 |
| Highest unidentified impurity | < 0.01 | < 0.01 | 0.019 | 0.017 | 0.011 | 0.018 |
| All impurities | 0.027 | 0.027 | 0.16 | 0.132 | 0.221 | 0.207 |
| Polymorph form | P1 | P1 | P1 | P1 | P1 | P1 |

The stability of the composition is very good. The amount of unidentified impurities is lower than 0.01 % and the amount of all contaminants is less than 0.5% after 6 months storage in a bottle covered polyethylene cap at 40°C and at 75 % relative humidity. It is surprising to a person skilled in the art that the amount of the known contaminants, namely the free acid formed by hydrolysis from the active ingredient is less then 0.2 % notwithstanding that the composition contains a high amount of hygroscopic ingredient (approximately 60% of Prosolv HD 90).

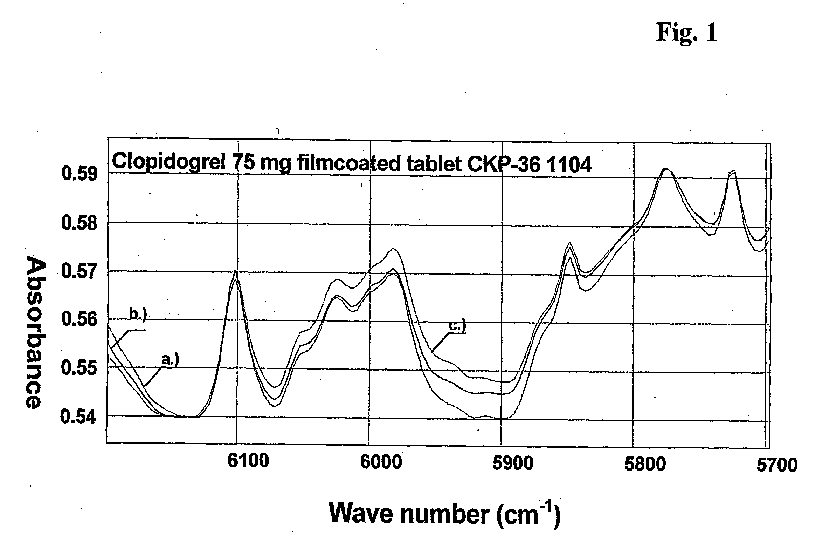

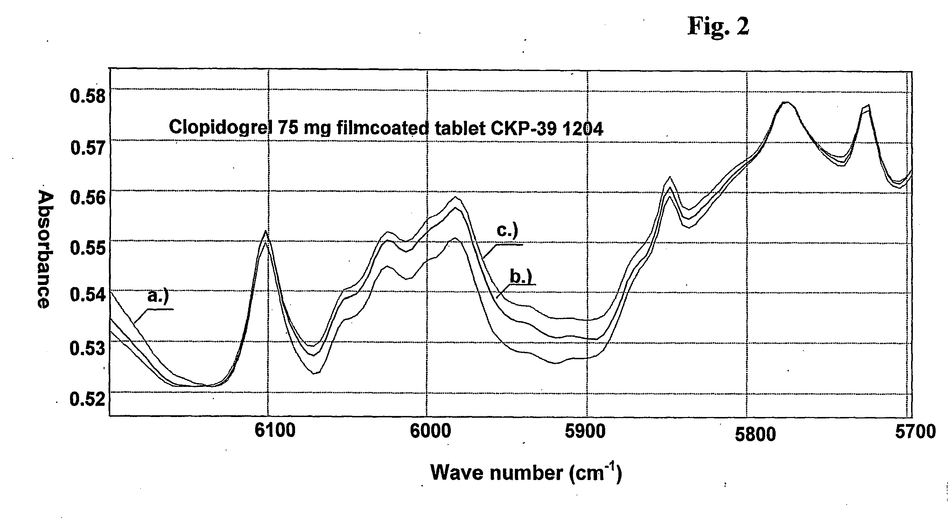

Surprisingly, the polymorph form of the active ingredient remains unchanged during the storage. It is proved by Figures 1 and 2, on which the NIR spectra of tablets are shown after the production and the long-term storages.

On Figure 1 the NIR spectra of tablets of batch No. 361104 and on Figure 2 the NIR spectra of tablets of batch No. 361204 are shown. These tablets are prepared according to Example 1 and contain clopidogrel hydrogensulphate of polymorph form 1.

On both Figures the spectra
a.) are measured at the time of production,
b.) are measured after 3 months long storage at 40°C and at 75 % relative humidity
c.) are measured after 6 months long storage at 40°C and at 75 % relative humidity.

The results of the examination of polymorphism show that both products prepared from the batches No. 361104 and No. 361204 are identical polymorph forms, namely polymorph form 1. There is no significant difference among the spectra, consequently the compositions are stable from the point of view of polymorphism too.

The mixture of microcrystalline cellulose and colloid silica has an advantageous effect on the dissolution properties of the active ingredient. As it is shown in Table 2, the dissolution rates of the tablets prepared from the mixture according to the present invention has not slowed down after a long time storage:

**Table 2**

| Dissolution data of tablets containing clopidogrel polymorph form 1: | | |
|---|---|---|
| Filmtablets according to the present invention | Dissolved active ingredient during 15 minutes | |
| | Before storage | 6 months at 40°C and at 75 % relative humidity |
| 361104 | 94,8% | 93,2% |
| 361204 | 94,5% | 97,1% |

In case of the composition according to the present invention no reduction of the dissolution rate takes place during the storage owing to the surprisingly suitable selection of the ingredients.

Based on the data shown above the chemical stability of compositions containing clopidogrel polymorph form 1 and a mixture of microcrystalline cellulose and colloid silica as binding and filling agent meet the rigorous requirements of pharmacopoeias.

Particularly, the present invention provides a solid dosage form which contains 20-40 % by weight, preferably 25-35 % by weight of clopidogrel hydrogensulphate of polymorph form 1, 40-65 % by weight, preferably 45-65 % by weight, more preferably 55-65 % by weight of microcrystalline cellulose, 0.5-10 % by weight, preferably 1-5 % by weight colloid silica, 3-20 % by weight, preferably 3-10 % by weight, more preferably 3-8 % by weight of disintegrating agent and 1-5 % by weight of lubricant based on the total weight of the composition.

According to the present invention a composite compound prepared from microcrystalline cellulose and colloid silica can be used instead of a mixture of microcrystalline cellulose and colloid silica.

The solid dosage forms according to the present invention are tablets or filmcoated tablets.

The filmcoated tablets contain 2-5 % by weight of water soluble polymer film on the surface of the tablet.

According to the most preferable embodiment of the present invention the composition contains microcrystalline cellulose having a grain size larger than 90 µm as microcrystalline cellulose; Prosolv HD 90 as siliconized microcrystalline cellulose, colloid silica having larger than 150 m²/g preferably larger than 200 m²/g specific surface as gliding agent, low substituted hydroxypropylcellulose as disintegrating agent and hydrogenated castor oil as lubricant.

Further embodiment of the present invention is a process for the preparation of a pharmaceutical composition wherein clopidogrel hydrogensulphate polymorph form 1, microcrystalline cellulose, colloid silica or a composite made of microcrystalline cellulose and colloid silica or the mixtures thereof, a disintegrating agent and a lubricant are mixed, homogenized, and the mixture is formed to a galenic form. In case of the use of colloid silica, the colloid silica is homogenized with a part of microcrystalline cellulose and sieved.

The preparation of the solid dosage forms according to the present invention, tablets, filmcoated tablets or capsules containing clopidogrel hydrogensulphate polymorph form 1 and the ingredients listed above can be carried out with the usual processes. Optimization of the process is a part of the knowledge of a person skilled in the art.

In course of the formation of the galenic form the powder mixture is pressed to tablets and if necessary coated with water soluble coating, or the powder mixture is filled into capsules.

According to the most preferable embodiment of the present invention, based on the weight of the composition 25-35 % by weight of clopidogrel hydrogensulphate of polymorph form 1 is mixed with 55-65 % by weight of microcrystalline cellulose, 1-5 % by weight, preferably colloid silica, or with 55-65 % by weight of the composite compound containing microcrystalline cellulose and colloid silica or a mixture thereof, 3-8 % by weight of low substituted hydroxypropylcellulose and 1-5 % by weight of hydrogenated castor oil, the mixture is homogenized and filled into capsules or the homogenizate is compressed to tablets using a direct compressing process and if necessary the obtained tablets are applied with water soluble coating.

During the development to produce a stable tablet containing clopidogrel polymorph form 1 a lot of experiments were carried out. The dissolution rate was fast in case of using polyvinyl pyrrolidone as binding agent, but the tablets changed colour after some days storage and the active ingredient decomposed.

In case of the tablets containing mannitol, hydroxypropyl cellulose and hydrogenated castor oil (Cutina H) the stability of the polymorph form of clopidogrel hydrogensulphate was not appropriate. The polymorph form 1 partly transformed to polymorph form 2 in the composition after two weeks storage, moreover the chemical decomposition of the active ingredient and the decrease of dissolution rate could be observed

In the course of the further examinations we found surprisingly that in the powder mixtures of clopidogrel hydrogensulphate of polymorph form 1 with microcrystalline cellulose and colloid silica the chemical and crystallographic stability of the active ingredient is appropriate and the dissolution rate of the tablets prepared from these powder mixtures is changeless during storage.

The advantageous effects, the chemical and crystallographic stability and the appropriate dissolution rate are demonstrated by not only the physical mixture of microcrystalline cellulose and colloid silica but by the composite substances made of microcrystalline cellulose and colloid silica as well. These substances are the so-called siliconized microcrystalline celluloses, which are marketed under the trademark Prosolv^{®}. These composites contain 98% of microcrystalline cellulose and 2% of colloid silica.

The microcrystalline cellulose and the siliconized microcrystalline cellulose are used as binding and filling agents.

For the preparation of the pharmaceutical composition containing clopidogrel hydrogensulphate of polymorph form 1 all types of microcrystalline cellulose or siliconized microcrystalline cellulose can be used, which have 90 µm or a larger grain size and appropriate flowability properties. From among siliconized microcrystalline celluloses the Prosolv^{®} HD 90 having a higher mass per volume is preferred.

Any colloid silica type, applicable in the pharmaceutical industry, can be used as a flowability enhancer, independently of the volume per mass or the specific surface thereof. It is advantageous to use such types of colloid silica, which are generally used for the preparation of capsules or tablets, having a specific surface of 200 m²/g.

In addition to the filling and binding agents the pharmaceutical compositions according to the present invention may contain further auxiliary agents as disintegration agents and lubricants.

In the course of selection of the disintegrating agents and lubricants the use of salt type compounds should be avoided because they may expedite the decomposition of the active ingredient. Fatty acids, e.g. stearic acid or fatty acid esters e.g. glyceryl behanate (Compritol 888), hydrogenated vegetable oils (e.g. Lubritab) or a hydrogenated castor oil (Cutina H) can be used as lubricant. According to our examinations the use of a hydrogenated castor oil (Cutina H) is the most preferable from the point of view of compressibility and chemical stability.

As disintegrating agent different types of starches, e.g. natural or pregelatinized starch, cross-linked polyvinylpyrrolidone (crospovidone) and low substituted hydroxypropyl cellulose (L-HPC) can be used. According to our examinations the use of L-HPC is the most preferable.

The disintegrating agents and lubricants are used preferably in a quantity of 3-20 % by weight and 1-5 % by weight respectively, based on the total weight of the composition, as it is usual in the processes for the preparation of tablets or capsules.

For the coating of the filmcoated tablets hydroxy-propyl-methyl- cellulose, polyvinyl alcohol or polyvinyl alcohol polyethylene glycol copolymer can be used. In this case the coating does not delay the dissolution of the active ingredient. For the preparation of the film coating the suspension can be prepared by the dissolution or suspension of the necessary components, namely the coating polymer, plasticizer and the pigment dyes, but ready-to-use complete coating systems (e.g. Opadry, Sepifilm, Aquapolish) can also be applied.

In case of the preparation of capsules the powder mixture containing the active ingredient and auxiliary agents is filled in hard gelatine or hydroxy-propyl-methyl-cellulose capsules.

The advantages of the pharmaceutical compositions according to the present invention are that
- using a powder mixing process and thereafter a direct tablet compressing or capsule filling process a pharmaceutical composition can be achieved containing clopidogrel hydrogensulphate of the polymorph form 1, in which
- the active ingredient keeps it polymorph form and chemical stability for a long time,
- the amount of all contaminants is less than 0.5% and
- the dissolution rate does not decrease substantially compared to the initial dissolution even after a long time storage, and after 15 minutes it is higher than 90%, which considerably exceeds the expected value of 80%.

Further details of the present invention are to be found in the following Examples without limiting the scope of protection to said Examples.

### Example 1

Into a 90 1 homogenizing equipment 9.79 kg of clopidogrel hydrogenesulphate of polymorph form 1 , 19.92 kg of siliconized microcrystalline cellulose (Prosolv HD 90), 1. 2 kg of L-HPC B1 and 1.2 kg of hydrogenated castor oil (Cutina H) are added. The mixture is homogenized with 25 rpm for 20 minutes, then pressed into biconvex tablets weighing 320 mg. Onto the obtained tablets a coating is applied, having a base of hydroxy-propyl-methylcellulose (Pharmacoat 606). The coating agent Opadry containing Pharmacoat 606 is dispersed in 10 % by weight in water in an amount enough to provide a coating layer of 10 mg/tablets. The coating suspension is sprayed onto the surface of tablets using 40-45°C bed temperature.

The process was repeated to examine the reproducibility. It is shown in Table 1 above what is the contaminant content of the product prepared according to this process after the preparation and what it is after a long time storage. The crystallographic properties of these products are shown on Figures 1 and 2. The dissolution rates are demonstrated in Table 2 above.

### Example 2

Into a drum mixer 0.5 kg of colloid silica of Aerosil 200 and 5.0 kg of microcrystalline cellulose of Avicel PH 102 are added. The mixture is homogenized and sieved through a sieve having a screen of 0.5 mm. The sieved homogenizate thus obtained, 9.79 kg of clopidogrel hydrogensulphate of polymorph form 1, 14.42 kg of microcrystalline cellulose (Avicel PH 102), 1. 2 kg of L-HPC B1 and 1.2 kg of hydrogenated castor oil (Cutina H) are added into a 901 homogenizing equipment. The mixture is homogenized with 25 rpm for 20 minutes, then pressed into biconvex tablets weighing 320 mg and having a diameter of 10 mm.

Onto the obtained tablets a coating is applied having a base of hydroxy-propyl-methylcellulose (Pharmacoat 606). The coating agent Opadry containing Pharmacoat 606 is dispersed in 10 % by weight in water in an amount enough to provide a coating layer of 10 mg/tablets. The coating suspension is sprayed onto the surface of tablets using 40-45°C bed temperature.

## Claims

1. Solid pharmaceutical dosage form containing polymorph form 1 of clopidogrel hydrogensulphate of the formula 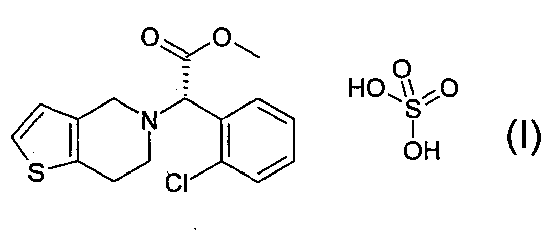 as active ingredient, microcrystalline cellulose and colloid silica as filling and binding agent, a disintegrating agent and a lubricant as further excipients.

2. Solid pharmaceutical dosage form according to Claim 1 **characterized in that** the pharmaceutical composition contains, based on its total weight, 20-40 % by weight, preferably 25-35 % by weight of clopidogrel hydrogensulphate of polymorph form 1, 40-65 % by weight, preferably 45-65 % by weight, more preferably 55-65 % by weight of microcrystalline cellulose, 0.5-10 % by weight, preferably 1-5 % by weight colloidal silica, 3-20 % by weight, preferably 3-10 % by weight, more preferably 3-8 % by weight of disintegrating agent and 1-5 % by weight of lubricant.

3. Solid pharmaceutical dosage form according to Claim 1 **characterized in that** the pharmaceutical composition contains, based on its total weight, 25-35 % by weight of the composition of clopidogrel hydrogensulphate of polymorph form 1, 55-65 weight % of microcrystalline cellulose, 1-5 % by weight of colloid silica, 3-8 % by weight of low substituted hydroxypropyl cellulose as disintegrant and 1-5 % by weight of hydrogenated castor oil as lubricant.

4. Solid pharmaceutical dosage form according to Claim 1 **characterized in that** the pharmaceutical composition contains, based on its total weight, 20-40 % by weight, preferably 25-35 % by weight of clopidogrel hydrogensulphate of polymorph form 1, 40-65 % by weight, preferably 45-65 % by weight, more preferably 55-65 % by weight of a composite substance made of microcrystalline cellulose and colloidal silica, 3-8 % by weight, preferably 3-10 % by weight, more preferably 3-8 % by weight of low substituted hydroxypropyl cellulose as disintegrating agent and 1-5 % by weight of hydrogenated castor oil as lubricant.

5. Solid pharmaceutical dosage form according to Claim 1 **characterized in that** the pharmaceutical composition contains, based on its total weight, 25-35 % by weight of the composition of clopidogrel hydrogensulphate of polymorph form 1, 55-65 % by weight of composite substance made of microcrystalline cellulose and colloidal silica, 3-8 % by weight of low substituted hydroxypropyl cellulose as disintegrating agent and 1-5 % by weight of hydrogenated castor oil as lubricant.

6. Pharmaceutical composition according any of Claims 1-5 **characterized in that** the formula is tablet, filmcoated tablet or capsule, preferably filmcoated tablet.

7. Filmcoated tablet according to Claim 6 **characterized in that** 2-5 % by weight of water soluble coating is applied on the basis of the total weight of the composition.

8. Pharmaceutical composition according to any of Claims 1-7 **characterized in that** the used microcrystalline cellulose has a grain size higher than 90 µm or the used siliconized microcrystalline cellulose is Prosolv^{®} HD 90.

9. Pharmaceutical composition according to any of Claims 1-8 **characterized in that** the used flowability enhancing agent, if necessary, is a colloidal silica having a specific surface of 150 m²/g, preferably 200 m²/g.

10. Pharmaceutical composition according to any of Claims 1-9 **characterized in that** the used disintegrating agent is selected from natural or pregelatinized starch, crosslinked polyvinylpyrrolidone or low substituted hydroxypropyl cellulose, preferably low substituted hydroxypropyl cellulose.

11. Pharmaceutical composition according to any of Claims 1-10 **characterized in that** the used lubricant is selected from fatty acids, fatty acid esters, hydrogenated vegetable oils, preferably hydrogenated castor oil.

12. Process for the preparation of a pharmaceutical composition according to any of Claims 1-11 **characterized in that** clopidogrel hydrogensulphate of polymorph 1 form, microcrystalline cellulose, colloidal silica or a composite substance made of microcrystalline cellulose and colloidal silica, or a mixture thereof is mixed with a disintegrating agent and a lubricant, the obtained mixture is homogenized and formed into a solid dosage form.

13. Process according to Claim 12 **characterized in that** the homogenized powder mixture is compressed to tablets by means of a direct tablet compressing process and the tablets are coated with a water soluble coating, if necessary.

14. Process according to Claim 12 **characterized in that** the homogenized powder mixture is filled in capsules.

15. Process for the preparation of the pharmaceutical compositions according to Claim 6 **characterized in that** based on the weight of the pharmaceutical composition 25-35 % by weight of clopidogrel hydrogensulphate of polymorph form 1, 55-65 % by weight of microcrystalline cellulose, 1-5 % by weight of colloidal silica, or 55-65 % by weight of composite compound of microcrystalline cellulose and colloidal silica, 3-8 % by weight of low substituted hydroxypropylcellulose and 1-5 % by weight of hydrogenated castor oil are mixed, homogenized, the obtained homogenizate is filled into capsules or pressed to tablets by means of a direct compressing process and the tablets are coated with a
water soluble coating, if necessary.

## Patentansprüche

1. Feste pharmazeutische Dosierungsform, enthaltend die polymorphe Form 1 von Clopidogrelhydrogensulfat der Formel 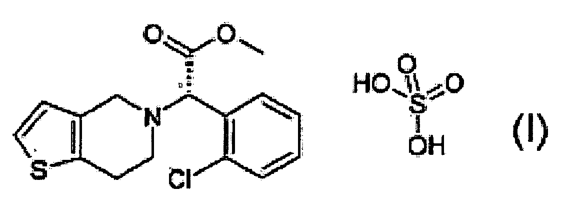 als Wirkstoff, mikrokristalline Cellulose und kolloidales Silica als Füll- und Bindemittel, ein Zerfallsmittel und ein Gleitmittel als weitere Hilfsstoffe.

2. Feste pharmazeutische Dosierungsform gemäß Anspruch 1, **dadurch gekennzeichnet, dass** die pharmazeutische Zusammensetzung, bezogen auf ihr Gesamtgewicht, 20 - 40 Gew.-%, vorzugsweise 25 - 35 Gew.-% Clopidogrelhydrogensulfat der polymorphen Form 1, 40 - 65 Gew.-%, vorzugsweise 45 - 65 Gew.-%, stärker bevorzugt 55 - 65 Gew.-% mikrokristalline Cellulose, 0,5 - 10 Gew.-%, vorzugsweise 1 - 5 Gew.-% kolloidales Silica, 3 - 20 Gew.-%, vorzugsweise, 3 - 10 Gew.-%, stärker bevorzugt 3 - 8 Gew.-% Zerfallsmittel und 1 - 5 Gew.-% Gleitmittel enthält.

3. Feste pharmazeutische Dosierungsform gemäß Anspruch 1, **dadurch gekennzeichnet, dass** die pharmazeutische Zusammensetzung, bezogen auf ihr Gesamtgewicht, 25 - 35 %, bezogen auf das Gewicht der Zusammensetzung, Clopidogrelhydrogensulfat der polymorphen Form 1, 55 - 65 Gew.% mikrokristalline Cellulose, 1 - 5 Gew.-% kolloidales Silica, 3 - 8 Gew.-% niedrig substituierte Hydroxypropylcellulose als Zerfallsmittel und 1 - 5 Gew.-% hydriertes Castoröl als Gleitmittel enthält.

4. Feste pharmazeutische Dosierungsform gemäß Anspruch 1, **dadurch gekennzeichnet, dass** die pharmazeutische Zusammensetzung, bezogen auf ihr Gesamtgewicht, 20 - 40 Gew.-%, vorzugsweise 25 - 35 Gew.-% Clopidogrelhydrogensulfat der polymorphen Form 1, 40 - 65 Gew.-%, vorzugsweise 45 - 65 Gew.-%, stärker bevorzugt 55 - 65 Gew.-% einer Verbundsubstanz aus mikrokristalliner Cellulose und kolloidalem Silica, 3 - 8 Gew.-%, vorzugsweise 3 - 10 Gew.-%, stärker bevorzugt 3 - 8 Gew.-% niedrig substituierte Hydroxypropylcellulose als Zerfallsmittel und 1 - 5 Gew.-% hydriertes Castoröl als Gleitmittel enthält.

5. Feste pharmazeutische Dosierungsform gemäß Anspruch 1, **dadurch gekennzeichnet, dass** die pharmazeutische Zusammensetzung, bezogen auf ihr Gesamtgewicht, 25 - 35 %, bezogen auf das Gewicht der Zusammensetzung, Clopidogrelhydrogensulfat der polymorphen Form 1, 55 - 65 Gew.-% einer Verbundsubstanz aus mikrokristalliner Cellulose und kolloidalem Silica, 3 - 8 Gew.-% niedrig substituierte Hydroxypropylcellulose als Zerfallsmittel und 1 - 5 Gew.-% hydriertes Castoröl als Gleitmittel enthält.

6. Pharmazeutische Zusammensetzung gemäß einem beliebigen der Ansprüche 1 - 5, **dadurch gekennzeichnet, dass** die Formulierung eine Tablette, eine filmbeschichtete Tablette oder Kapsel, vorzugsweise eine filmbeschichtete Tablette ist.

7. Filmbeschichtete Tablette gemäß Anspruch 6, **dadurch gekennzeichnet, dass** 2 - 5 Gew.-% wasserlösliche Beschichtung, bezogen auf das Gesamtgewicht der Zusammensetzung, aufgetragen werden.

8. Pharmazeutische Zusammensetzung gemäß einem beliebigen der Ansprüche 1 - 7, **dadurch gekennzeichnet, dass** die verwendete mikrokristalline Cellulose eine höhere Korngröße als 90 µm aufweist oder die verwendete siliconisierte Mikrocellulose Prosolv^{®} HD 90 ist.

9. Pharmazeutische Zusammensetzung gemäß einem beliebigen der Ansprüche 1 - 8, **dadurch gekennzeichnet, dass** das die Fließfähigkeit erhöhende Mittel, sofern erforderlich, ein kolloidales Silica mit einer spezifischen Oberfläche von 150 m²/g, vorzugsweise 200 m²/g, ist.

10. Pharmazeutische Zusammensetzung gemäß einem beliebigen der Ansprüche 1 - 9, **dadurch gekennzeichnet, dass** das verwendete Zerfallsmittel aus natürlicher oder vorgelatinisierter Stärke, quervernetztem Polyvinylpyrrolidon oder niedrig substituierter Hydroxypropylcellulose, vorzugsweise niedrig substituierter Hydroxypropylcellulose, ausgewählt ist.

11. Pharmazeutische Zusammensetzung gemäß einem beliebigen der Ansprüche 1 - 10, **dadurch gekennzeichnet, dass** das verwendete Gleitmittel aus Fettsäuren, Fettsäureestern, hydrierten Pflanzenölen, vorzugsweise hydriertem Castoröl, ausgewählt ist.

12. Verfahren zur Herstellung einer pharmazeutischen Zusammensetzung gemäß einem beliebigen der Ansprüche 1 - 11, **dadurch gekennzeichnet, dass** das Clopidogrelhydrogensulfat der polymorphen Form 1, mikrokristalline Cellulose, kolloidales Silica oder eine Verbundsubstanz aus mikrokristalliner Cellulose und kolloidalem Silica oder eine Mischung davon mit einem Zerfallsmittel und einem Gleitmittel gemischt werden, die erhaltene Mischung homogenisiert und in eine feste Dosierungsform gebracht wird.

13. Verfahren gemäß Anspruch 12, **dadurch gekennzeichnet, dass** die homogenisierte Pulvermischung zu Tabletten mittels eines direkten Tablettenpressverfahrens gepresst wird und die Tabletten, sofern erforderlich, mit einer wasserlöslichen Beschichtung überzogen werden.

14. Verfahren gemäß Anspruch 12, **dadurch gekennzeichnet, dass** die homogenisierte Pulvermischung in Kapseln gefüllt wird.

15. Verfahren zur Herstellung der pharmazeutischen Zusammensetzungen gemäß Anspruch 6, **dadurch gekennzeichnet dass**, bezogen auf das Gewicht der pharmazeutischen Zusammensetzung, 25 - 35 Gew.-% Clopidogrelhydrogensulfat der polymorphen Form 1, 55 - 65 Gew.-% mikrokristalline Cellulose, 1 - 5 Gew.-% kolloidales Silica, oder 55 - 65 Gew.-% an Verbundverbindung aus mikrokristalliner Cellulose und kolloidalem Silica, 3 - 8 Gew.-% an niedrig substituierter Hydroxypropylcellulose und 1 - 5 Gew.-% an hydriertem Castoröl gemischt werden, homogenisiert werden, das Homogenisat in Kapseln gefüllt oder zu Tabletten mit Hilfe eines direkten Pressverfahrens gepresst wird, und die Tabletten, sofern erforderlich, mit einer wasserlöslichen Beschichtung überzogen werden.

## Revendications

1. Forme pharmaceutique posologique solide contenant une forme polymorphe 1 d'hydrogénosulfate de clopidogrel de formule 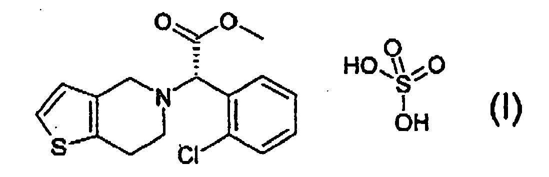 en tant qu'ingrédient actif, de la cellulose microcristalline et de la silice colloïdale en tant qu'agent de charge et liant, un agent désintégrant et un lubrifiant en tant qu'excipients supplémentaires.

2. Forme pharmaceutique posologique solide selon la revendication 1, **caractérisée en ce que** la composition pharmaceutique contient, par rapport à son poids total, 20 à 40 % en poids, de préférence 25 à 35 % en poids d'hydrogénosulfate de clopidogrel de forme polymorphe I, 40 à 65 % en poids, de préférence 45 à 65 % en poids, de préférence encore 55 à 65 % en poids de cellulose microcristalline, 0,5 à 10 % en poids, de préférence 1 à 5 % en poids de silice colloïdale, 3 à 20 % en poids, de préférence 3 à 10 % en poids, de préférence encore 3 à 8 % en poids d'agent désintégrant et 1 à 5 % en poids de lubrifiant.

3. Forme pharmaceutique posologique solide selon la revendication 1, **caractérisée en ce que** la composition pharmaceutique contient, par rapport à son poids total, 25 à 35 %, en poids de la composition, d'hydrogénosulfate de clopidogrel de forme polymorphe I, 55 à 65 % en poids de cellulose microcristalline, 1 à 5 % en poids de silice colloïdale, 3 à 8 % en poids d'hydroxypropylcellulose faiblement substituée en tant que désintégrant et 1 à 5 % en poids d'huile de ricin hydrogénée en tant que lubrifiant.

4. Forme pharmaceutique posologique solide selon la revendication 1, **caractérisée en ce que** la composition pharmaceutique contient, par rapport à son poids total, 20 à 40 % en poids, de préférence 25 à 35 % en poids d'hydrogénosulfate de clopidogrel de forme polymorphe I, 40 à 65 % en poids, de préférence 45 à 65 % en poids, de préférence encore 55 à 65 % en poids d'une substance composite faite de cellulose microcristalline et de silice colloïdale, 3 à 8 % en poids, de préférence 3 à 10 % en poids, de préférence encore 3 à 8 % en poids d'hydroxypropylcellulose faiblement substituée en tant qu'agent désintégrant et 1 à 5 % en poids d'huile de ricin hydrogénée en tant que lubrifiant.

5. Forme pharmaceutique posologique solide selon la revendication 1, **caractérisée en ce que** la composition pharmaceutique contient, par rapport à son poids total, 25 à 35 %, en poids de la composition, d'hydrogénosulfate de clopidogrel de forme polymorphe I, 55 à 65 % en poids d'une substance composite faite de cellulose microcristalline et de silice colloïdale, 3 à 8 % en poids d'hydroxypropylcellulose faiblement substituée en tant qu'agent désintégrant et 1 à 5 % en poids d'huile de ricin hydrogénée en tant que lubrifiant.

6. Composition pharmaceutique selon l'une quelconque des revendications 1 à 5, **caractérisée en ce que** la formule est un comprimé, un comprimé pelliculé ou une capsule, de préférence un comprimé pelliculé.

7. Comprimé pelliculé selon la revendication 6, **caractérisé en ce que** 2 à 5 % en poids d'enrobage hydrosoluble est appliqué par rapport au poids total de la composition.

8. Composition pharmaceutique selon l'une quelconque des revendications 1 à 7, **caractérisée en ce que** la cellulose microcristalline utilisée a une taille de grain supérieure à 90 µm ou la cellulose microcristalline siliconisée utilisée est le Prosolv® HD 90.

9. Composition pharmaceutique selon l'une quelconque des revendications 1 à 8, **caractérisée en ce que** l'agent amplifiant la fluidité utilisé, si nécessaire, est une silice colloïdale ayant une surface spécifique de 150 m²/g, de préférence 200 m²/g.

10. Composition pharmaceutique selon l'une quelconque des revendications 1 à 9, **caractérisée en ce que** l'agent désintégrant utilisé est choisi parmi l'amidon naturel ou prégélatinisé, une polyvinylpyrrolidone réticulée ou une hydroxypropylcellulose faiblement substituée, de préférence une hydroxypropylcellulose faiblement substituée.

11. Composition pharmaceutique selon l'une quelconque des revendications 1 à 10, **caractérisée en ce que** le lubrifiant utilisé est choisi parmi les acides gras, les esters d'acides gras, les huiles végétales hydrogénées, de préférence l'huile de ricin hydrogénée.

12. Procédé pour la préparation d'une composition pharmaceutique selon l'une quelconque des revendications 1 à 11, **caractérisé en ce que** l'hydrogénosulfate de clopidogrel de forme polymorphe 1, la cellulose microcristalline, la silice colloïdale ou une substance composite faite de cellulose microcristalline et de silice colloïdale, ou un mélange de ceux-ci, est mélangé(e) avec un agent désintégrant et un lubrifiant, le mélange obtenu est homogénéisé et façonné en une forme posologique solide.

13. Procédé selon la revendication 12, **caractérisé en ce que** le mélange de poudres homogénéisé est compressé en comprimés au moyen d'un procédé de compression directe de comprimés et les comprimés sont enrobés avec un enrobage hydrosoluble, si nécessaire.

14. Procédé selon la revendication 12, **caractérisé en ce que** le mélange de poudres homogénéisé est introduit dans des capsules.

15. Procédé pour la préparation des compositions pharmaceutiques selon la revendication 6, **caractérisé en ce que**, par rapport au poids de la composition pharmaceutique, 25 à 35 % en poids d'hydrogénosulfate de clopidogrel de forme polymorphe I, 55 à 65 % en poids de cellulose microcristalline, 1 à 5 % en poids de silice colloïdale, ou 55 à 65 % en poids d'un composé composite de cellulose microcristalline et de silice colloïdale, 3 à 8 % en poids d'hydroxypropylcellulose faiblement substituée et 1 à 5 % en poids d'huile de ricin hydrogénée sont mélangés, homogénéisés, l'homogénat obtenu est introduit dans des capsules ou pressé en comprimés au moyen d'un procédé de compression directe et les comprimés sont enrobés avec un enrobage hydrosoluble, si nécessaire.
